(19) Europäisches Patentamt
European Patent Office
Office européen des brevets

(11) Publication number: **0 130 013**
**A2**

# (12) EUROPEAN PATENT APPLICATION

(21) Application number: 84304025.4

(22) Date of filing: 14.06.84

(51) Int. Cl.[4]: C 01 B 33/20
B 01 J 29/04, C 01 B 33/28

(30) Priority: 14.06.83 GB 8316168

(43) Date of publication of application: 02.01.85 Bulletin 85/1

(84) Designated Contracting States: BE DE FR GB IT NL

(71) Applicant: The British Petroleum Company p.l.c.
Britannic House Moor Lane
London EC2Y 9BU(GB)

(72) Inventor: Barri, Sami Ali Ibrahim
The British Petroleum Company p.l.c. Chertsey Road
Sunbury-on-Thames Middlesex, TW16 7LN(GB)

(72) Inventor: Young, Dennis
The British Petroleum Company p.l.c. Chertsey Road
Sunbury-on-Thames Middlesex, TW16 7LN(GB)

(74) Representative: Krishnan, Suryanarayana Kalyana et al,
c/o The British Petroleum Company plc Patents Division
Chertsey Road
Sunbury-on-Thames Middlesex TW16 7LN(GB)

## (54) Crystalline gallo-silicates, a process for producing them and their use as catalysts.

(57) The present invention relates to crystalline gallosilicates having a mordenite type structure, to methods of preparing the same, and the use thereof as catalysts in hydrocarbon conversion reactions. The gallosilicates have different acidity properties to the analogous MOR-type conventional aluminosilicates and can be produced by crystallisation from a mixture containing gallia, silica, alkali metal(s), water and a template. The gallosilicates can be used, whether or not impregnated and/or ion-exchanged, admixed, supported or bound as catalysts for any of the following reactions: alkylation, dealkylation, dehydrocyclodimerisation, aromatisation, transalkylation, isomerisation, dehydrogenation, hydrogenation, cracking, hydrocracking, cyclisation, oligomerisation, polymerisation, and dehydration reactions, or for conversion of carbon monoxide/hydrogen mixtures to hydrocarbons.

# CRYSTALLINE GALLO-SILICATES, A PROCESS FOR PRODUCING THEM AND THEIR USE AS CATALYSTS

The present invention relates to novel gallosilicates and to methods of preparing the same. More particularly, this invention relates to novel crystalline gallosilicates having a mordenite type structure, to methods of preparing the same, and the use thereof as catalysts in hydrocarbon conversion reactions.

Zeolites are well known natural and synthetic compositions. Many of them have been demonstrated to have catalytic properties for various types of hydrocarbon and related reactions. Zeolites can be defined as ordered porous crystalline aluminosilicates having a framework structure sufficiently open to accommodate at least water molecules. Such structures generally contain a regular array of small voids interconnected by channels or pores. The dimensions of the voids and channels can range from those of water to those of quite large molecules. For a given framework structure, the dimensions of the voids and channels are limited to a small number of values, which can vary from structure to structure. Thus these structures are capable of absorbing molecules of certain dimensions while rejecting those of dimensions larger than a critical value which varies with structure. This has led to zeolites being used as molecular sieves. Zeolites belong to a class of materials that can be termed tectoaluminosilicates which comprise (in addition to zeolites) felspars and felspathoids. They can be defined as having a framework structure consisting of a rigid regular three dimensional network of $SiO_4$ and $AlO_4$ tetrahedra in which the tetrahedra are cross-linked by sharing the oxygen atoms. All oxygen

atoms are shared, thus the ratio of total aluminium and silicon atoms to oxygen atoms is 1:2. The inclusion of aluminium in the framework leads to a net negative charge which is balanced by the inclusion in the crystal of an electrochemical equivalence of cations, for example alkali metal, alkaline earth metal, hydrogen or ammonium cations or mixtures thereof. This can be expressed by a formula in which the ratio of Al to the number of the various cations such as Ca/2, Sr/2, Na, K, Li or generally M/n (where n is the normal oxidation state of the cation) is equal to unity. Additionally in zeolites, but not in felspars and some felspathoids, the framework is sufficiently open to accommodate water molecules as well as cations. This enables these cations to be exchanged in their entirety or partially by other cations using ion-exchange techniques in a conventional manner. These materials can exhibit specific affinities for specific cations and can thus be used as selective ion-exchangers. By means of ion-exchange, it is possible to vary the size of the pores in a given crystalline zeolite material, modifying its molecular sieve properties. Also by means of ion-exchange the catalytic properties of these materials can be altered. In addition to the framework and charge-compensating cations, zeolites can contain other materials such as water and organic molecules, (hydrated) salts and oxides of eg Na, Al and Si introduced during synthesis, or formed or added during subsequent treatments. Zeolites are best characterised according to framework structure type, ie the topology of the framework, irrespective of composition, distribution of different tetrahedral atoms, cell dimensions and symmetry. A code consisting of three capital letters has been adopted for each known structure type following the recommendations by IUPAC on zeolite nomenclature ("Chemical Nomenclature, and Formulation of Compositions, of Synthetic and Natural Zeolites," IUPAC yellow booklet, 1978) and a compilation of 38 known zeolite structure types has been published by The Structure Commission of the International Zeolite Association ("Atlas of Zeolite Structure Types", by Meier, W.M. and Olsen, D.H. (1978), distributed by Polycrystal Book Service, Pittsburgh, Pa, USA). In

addition to the groups classified by known structure type, there is a further group of crystalline zeolite materials whose X-ray diffraction patterns, sorption, ion-exchange and related properties indicate that they do not have known structure types but appear to have new, as yet undetermined structure types. An example of such a material is the novel porous crystalline aluminosilicate designated Theta-1 and described in our published copending European patent specification No 0057049.

Our copending European application published under No. 14023 describes a process for preparing crystalline aluminosilicates of the mordenite type, by crystallising from a mixture containing a source of silica, a source of alumina, a source of alkali metal and a nitrogenous organic base which is for instance a neopentylamine.

Zeolites (and other tectoaluminosilicates) belong to a larger class of materials that can be termed tectometallosilicates which can be defined in the same way as tectoaluminosilicates except that the aluminium is replaced by a range of elements, which includes, it is claimed, Ti, Zr, V, Cr, Mo, Mn, Fe, Co, Rh, Ni, Zn, B, Al, Ga, Ge, Sn, As and Sb, but in some cases the claimed materials have not been well characterised. In some cases (where the element has the same formal oxidation state as Si ie +4) the resultant framework is electro- neutral and the resultant materials resemble crystalline silicas. In other cases there is a resultant framework negative charge which must be compensated by cations as in tectoaluminosilicates. In some cases the materials have porous frameworks like those of zeolites or porous crystalline silicas which they therefore resemble.

A number of crystalline gallosilicates useful in catalytic applications have been previously disclosed. For example, gallosilicates having the following structure types (see "Atlas of Zeolite Structures" above) have been disclosed: FAU (USP 3431219), ANA (UK Patent Application GB 2102779A) and MFI (UK Patent Application GB 2053960A).

It has now been found that a novel crystalline gallosilicate, having a mordenite (MOR) type structure but with different acidity

properties to the analogous MOR-type aluminosilicates, can be produced by crystallisation from a mixture containing a source of gallia, a source of silica, a source of alkali metal(s), water and a template such as an organic nitrogen containing base.

Accordingly, the present invention provides novel crystalline gallosilicates having the following composition in terms of the mole ratios of the oxides:

$$0.9 \pm 0.25 M_{2/n}O : Ga_2O_3 : xSiO_2 : yH_2O : zQ$$

wherein M is at least one cation having a valence n, x is at least 3, y/x is from 0 to 5, Q is a template used in the synthesis of the gallosilicate and z is 0-20, and wherein the gallosilicate in the calcined hydrogen-form has an X-ray diffraction pattern substantially as set forth in Table A of this specification.

By the term "calcined hydrogen-form" is meant here and throughout this specification that the gallosilicate has been calcined to render it substantially free of the template introduced during synthesis or subsequent treatments, and the cation M is hydrogen. The hydrogen-form of the gallosilicate may also be produced by known methods such as exchange with hydrogen ions or with ammonium cations followed by one or more calcinations or a combination of the two followed by one or more calcination stages, if the gallosilicate still contained ammonium ions.

By "template" is meant throughout this specification a chemical agent which encourages crystallisation to proceed towards a particular framework structure or structures.

The $H_2O$ content "y" of the gallosilicate will depend, within the ratios set out above, upon the conditions under which it is dried, calcined, subjected to further aqueous treatments or combinations thereof after synthesis.

The content "z" of template "Q" in the gallosilicate will also depend upon the conditions under which it is washed, calcined or subjected to further aqueous treatments or combinations thereof after synthesis, and also on the synthesis parameters of the gallosilicate, particularly the proportion of Q present in the original hydrogel. The template content is usually highest for the

"parent" gallosilicate. Complete removal of the template is usually only possible by degradation of the template using a process involving thermal or oxidative reactions or both.

By the "parent" gallosilicate is meant throughout this specification the gallosilicate as synthesised and washed, with an optional drying step as hereinafter described.

The cation M in the gallosilicate may be selected from $H^+$, ammonium, alkali metal cations, alkaline earth metal cations, template containing cations, aluminium cations, gallium cation and mixtures thereof.

The cations present in the gallosilicate may be replaced using conventional ion exchange techniques either wholly or partially by other cations e.g. hydrogen ions or metal cations.

The gallosilicates according to the present invention have in their calcined hydrogen form an X-ray diffraction pattern shown in Table A below. The specific values in the Tables were determined using copper K-alpha radiation and a computer step scan.

The peak heights, I, and their position as a function of 2-theta, where theta is the Bragg angle, were read from the spectrometer output. From this output the relative intensities 100 x $I/I_o$, where $I_o$ is the intensity of the strongest peak, and d the interplanar spacing in Å, corresponding to the recorded peaks were calculated.

It will be understood by those skilled in the art that the X-ray diffraction pattern of gallosilicates may vary in the values of $I/I_o$ and the d-spacing depending for example upon whether the sample being examined is calcined or uncalcined, upon the temperature of calcination, upon the nature of the cation present in the gallosilicate, the mole ratio of silica to gallia, and the particle size of the gallosilicate.

The gallosilicate is suitably produced from an initial mixture containing a source of silica, a source of gallia, a source of alkali metal(s), water and a template such as an organic nitrogen containing base.

The silica to gallia mole ratio in the initial mixture is at

least 3:1. The silica to gallia mole ratio is suitably from 4:1 to 25:1 preferably in the range 8:1 to 20:1. The free alkali metal(s) hydroxide to water mole ratio, defined as:-

$$\frac{[(\text{Number of moles of total alkali metal(s)}) - (\text{Number of moles of alkali metal(s) required to convert gallia present to alkali metal gallate(s), ie } MGaO_2)]}{\text{Number of moles of water present}}$$

is greater than $1x10^{-5}$:1, suitably from $1x10^{-3}$:1 to $2x10^{-2}$:1, preferably in the range $2x10^{-3}$:1 to $8x10^{-3}$:1. Similarly the mole ratio of silica to free alkali metal(s) hydroxide may suitably be greater than 1, preferably from 2:1 to 500:1, most preferably from 5:1 to 100:1. The mole ratio of water to silica may suitably be less than 500:1, preferably from 6:1 to 30:1, even more preferably from 9:1 to 30:1.

In preparing the gel, sources of silica which may be used include for instance sodium silicate, silica hydrosol, silica gel, silica sol and silicic acid. It is preferable to use an aqueous colloidal dispersion of silica particles, eg the Ludox (Registered Trade Mark) varieties manufactured by DuPont.

The sources of gallia used may include for instance inorganic gallium salts such as the oxides, hydroxides and gallates. The source of gallium is preferably gallium hydroxide activated by the addition of aqueous alkali hydroxides and a nitrogen base to the appropriate proportion of freshly precipitated gallium hydroxide, and then the source of silica is slowly added to the activated gallium hydroxide.

The alkali metal(s) used in the initial mixture may similarly be the inorganic salts containing these metals, their oxides and their hydroxides. Sodium is the preferred alkali metal.

The template is suitably selected from one or more of the following:

(a) an alkene diamine or a dialkenetriamine or a polyalkylene polyamine which may be ethylene diamine, propylene diamine, butylene diamine, pentylene diamine, diethylene triamine, triethylene tetraamine, tetraethylene pentamine or pentaethylene hexamine, or the like,

(b) an alkanolamine which may be mono-, di- or tri-alkanol amine and in which the alkanol group is derived from ethanol, propanol or butanol,

(c) ammonia, and

(d) an alkylene glycol, a $C_1$-$C_6$ polyol or a polyoxyalkylene glycol in which the alkylene groups may be ethylene, propylene or butylene.

Of these, the templates referred to in (b) above are most preferred.

The gallosilicate is suitably prepared by forming a mixture of all the reactants, by simply mixing them together while maintaining the mixture suitably at a temperature from 0 to 100°C, preferably from 20 and 60°C, until a homogeneous gel is formed and crystallising the gel so-formed at a temperature above 70°C, preferably from 100 to 220°C for a period of at least 2 hours, preferably for 6 to 240 hours. The optimum crystallisation period can vary and may depend upon such factors as the temperature, agitation, pH, gel composition and seeding. The mixing and digestion of the ingredients is suitably carried out under autogenous pressure although the pressure can be further increased by pressurisation with a suitable gas, eg nitrogen. It is preferable to agitate the mixture during the crystallisation stages. It is preferable that the silica source is added to the other reagents in such a manner as to commence gelation at a relatively high pH within the range specified above.

The product obtained in this manner contains cations which may be hydrogen, alkali metal(s), gallium, or template cations or any combination thereof.

The cations in the product may be converted to hydrogen to give rise to the hydrogen-form of the product. This may be achieved by techniques known to those skilled in the art, e.g. (a) ammonia exchange followed by calcination, (b) acid exchange or a combination of (a) and (b).

The product or the hydrogen-form thereof may also be loaded with additional metals or oxides suitable for imparting a specific

type of catalytic activity. The loading may be achieved by conventional ion-exchange or impregnation techniques. The metal compounds which may be used for ion-exchange and/or impregnation may be compounds of any one of the following metals or groups of metals, namely those belonging to Groups IB, IIB, IIIA, IVA, VA, VIB, VIIB and VIII according to the Periodic Table due to Mendeleef. Specifically, compounds of copper,silver, zinc, aluminium, gallium, indium, thallium, lead, antimony, bismuth, chromium, molybdenum, tungsten, manganese, iron, cobalt, nickel, ruthenium, rhodium, palladium, iridium, platinum, rhenium, thorium and the rare earth metals are preferred.

The gallosilicate products of the present invention may be admixed or bound with other catalytic components or supports such as e.g. other zeolites or gallosilicates before or after impregnation or after exchange with one of the aforementioned metal compounds to produce an attrition resistant catalyst. The conventional alumina or silica binders may also be used.

The gallosilicates of the present invention may be used, whether or not impregnated and/or ion-exchanged, admixed, supported or bound as catalysts for any of the following reactions: alkylation, dealkylation, dehydrocyclodimerisation, aromatisation, transalkylation, isomerisation, dehydrogenation, hydrogenation, cracking, hydrocracking, cyclisation, oligomerisation, polymerisation, and dehydration reactions, particularly dehydration of alcohols and ethers. The gallosilicate catalysts may also be used for conversion of carbon monoxide/hydrogen mixtures to hydrocarbons. The gallosilicates may be used in the form of fixed, fluidised or moving beds.

The present invention is further illustrated with reference to the following Examples.

TABLE A

| 2-theta | d-spacing | 100 x I/Io |
|---|---|---|
| 6.33 - 6.73 | 13.95 - 13.12 | 30 - 60 |
| 8.44 - 8.84 | 10.47 - 9.99 | 5 - 15 |
| 9.55 - 9.99 | 9.25 - 8.85 | 50 - 80 |
| 13.25 - 13.65 | 6.68 - 6.48 | 50 - 80 |
| 13.65 - 14.05 | 6.48 - 6.30 | 10 - 25 |
| 14.39 - 14.79 | 6.15 - 5.98 | 10 - 25 |
| 15.09 - 15.49 | 5.87 - 5.72 | 10 - 25 |
| 17.37 - 17.77 | 5.10 - 4.99 | 2 - 10 |
| 17.99 - 18.39 | 4.92 - 4.82 | 1 - 5 |
| 19.40 - 19.80 | 4.57 - 4.48 | 20 - 40 |
| 21.22 - 21.62 | 4.18 - 4.11 | 2 - 10 |
| 22.05 - 22.45 | 4.03 - 3.96 | 50 - 80 |
| 22.98 - 23.38 | 3.87 - 3.80 | 10 - 30 |
| 23.41 - 23.81 | 3.80 - 3.73 | 5 - 15 |
| 24.30 - 24.70 | 3.66 - 3.60 | 5 - 15 |
| 24.67 - 25.07 | 3.61 - 3.55 | 2 - 10 |
| 24.97 - 25.37 | 5.56 - 3.51 | 5 - 20 |
| 25.39 - 25.79 | 3.51 - 3.45 | 80 - 100 |
| 26.07 - 26.47 | 3.42 - 3.36 | 35 - 65 |
| 26.84 - 27.24 | 3.32 - 3.27 | 2 - 10 |
| 27.38 - 27.78 | 3.25 - 3.21 | 45 - 85 |
| 27.64 - 28.04 | 3.22 - 3.18 | 25 - 50 |
| 28.02 - 28.42 | 3.18 - 3.14 | 2 - 10 |
| 28.48 - 28.88 | 3.13 - 3.09 | 2 - 10 |
| 29.46 - 29.86 | 3.03 - 2.99 | 5 - 15 |
| 30.63 - 31.03 | 2.92 - 2.88 | 20 - 35 |
| 32.43 - 32.83 | 2.76 - 2.73 | 2 - 10 |
| 32.94 - 33.34 | 2.72 - 2.68 | 2 - 10 |
| 34.34 - 34.74 | 2.61 - 2.58 | 1 - 5 |
| 34.82 - 35.22 | 2.57 - 2.55 | 2 - 10 |
| 35.27 - 35.67 | 2.54 - 2.51 | 10 - 20 |

Example 1

An aqueous solution of gallium nitrate containing 0.0235 moles of gallium was adjusted to a pH of about 6 using aqueous ammonia. The precipitate obtained was filtered and washed with distilled water and transferred into a beaker containing aqueous sodium hydroxide solution (55g, 0.0086 moles of sodium hydroxide). The mixture so formed was stirred until a clear solution was obtained. Diethanolamine (DEA) (40g) was melted and added to the gallate solution and the resultant solution "A" was stirred at 20°C for 10 minutes. 28g of a commercial silica gel, (Ludox AS40) (Reg. Trade Mark) which contains 40% by weight of silica, was further diluted with 112g of water and added to solution "A" over a period of 15 minutes with vigorous stirring which was continued for a further 20 minutes. The resultant gel, composition 3.66 $Na_2O$:32.4 DEA : $Ga_2O_3$:15.9 $SiO_2$:850 $H_2O$, was transferred to a stainless steel pressure vessel and crystallised at 175°C for 45 hours. The product was filtered, washed and dried at 90°C.

The product was found by X-ray powder diffraction to be substantially crystalline with a diffraction pattern (Table 1) very similar to and consistent with the mordenite structure. It contained Si (32%), Ga (16%) and Na (3.8%).

The product was also examined by $^{29}Si$ magic angle spinning NMR and the results are shown in Table 2 below.

TABLE 1

| 2-theta | d-spacing | 100 x I/Io |
|---|---|---|
| 6.53 | 13.52 | 44 |
| 8.64 | 10.23 | 8 |
| 9.75 | 9.06 | 67 |
| 13.45 | 6.58 | 69 |
| 13.85 | 6.39 | 17 |
| 14.59 | 6.07 | 15 |
| 15.29 | 5.79 | 18 |
| 17.57 | 5.04 | 4 |
| 18.19 | 4.87 | 2 |
| 19.60 | 4.53 | 32 |
| 21.42 | 4.14 | 6 |
| 22.25 | 3.992 | 67 |
| 23.18 | 3.835 | 20 |
| 23.61 | 3.766 | 8 |
| 24.50 | 3.631 | 7 |
| 24.87 | 3.577 | 4 |
| 25.17 | 3.535 | 11 |
| 25.59 | 3.478 | 100 |
| 26.27 | 3.389 | 53 |
| 27.04 | 3.295 | 6 |
| 27.58 | 3.232 | 58 |
| 27.84 | 3.203 | 36 |
| 28.22 | 3.160 | 6 |
| 28.68 | 3.110 | 7 |
| 29.56 | 2.941 | 8 |
| 30.83 | 2.898 | 27 |
| 32.63 | 2.742 | 4 |
| 33.14 | 2.701 | 6 |
| 34.54 | 2.594 | 3 |
| 35.02 | 2.560 | 5 |
| 35.57 | 2.522 | 15 |

Comparative Test

A solution "B" was prepared by melting and adding 42g of diethanolamine (DEA) to 56g of a clear solution containing 0.0141 moles of dissolved alumina and 0.0483 moles of dissolved sodium oxide, then stirring for 10 minutes. 28g of a commercial silica gel, (Ludox AS40) (Regd. Trade Mark) that contains 40% by weight of silica, was further diluted with 112g of water and added to solution "B" over a period of 15 minutes. The whole procedure was carried out at room temperature. The resultant gel, composition

$$3.43\ Na_2O:284\ DEA:Al_2O_3:13.2\ SiO_2:704\ H_2O,$$

was transferred to a stainless steel pressure vessel and crystallised at 175°C for 50 hours. The product was filtered, washed and dried at 90°C.

The product was found by X-ray powder diffraction to be a substantially crystalline sample of mordenite (as characterised eg by M.L. Sand, W.S. Coblenz and L.B. Sand, Molecular Sieve Zeolite, Advances in Chemistry Series, 101, American Chemical Society, Washington, D.C. 1971 p 127 and by R.M. Barrer, J. Chem. Soc. 1948, 2158-63). The sample was examined by $^{29}Si$ magic angle spinning NMR and gave signals as shown in Table 2.

TABLE 2

| Chemical Shift vs. Tetramethylsilane | | Assignment |
|---|---|---|
| Example | Comparative Test | |
| -112.3 | -112.3 | $Si(OSi)_4$ |
| -104.2 | | $Si(OSi)_3(OGa)$ |
| | -106.0 | $Si(OSi)_3(OAl)$ |
| - 97.1 | | $Si(OSi)_2(OGa)_2$ |
| | - 99.1 | $Si(OSi)_2(OAl)_2$ |

The d-spacing values in Table 1 are marginally higher than the corresponding published values for tectoaluminosilicates with a mordenite (MOR) type structure referred to in the Comparative Test. This is indicative of an expansion of the unit cell and is consistent with isomorphous substitution of aluminium by gallium in a MOR type structure.

In Table 2, the higher frequency values corresponding to chemical shifts for the product of the present invention (as produced by the Example) relative to the product of the Comparative Test indicate that the product of the present invention is a tectogallosilicate. This confirms the evidence of XRD data above that the product of the present invention is a tectogallosilicate of the MOR structure type.

Example 2

An aqueous solution of gallium nitrate containing 0.0113 moles of gallium was adjusted to a pH of about 6 using aqueous ammonia. The precipitate obtained was filtered and washed with distilled water and transferred to a beaker containing aqueous sodium hydroxide solution (45g with 0.0425 moles of sodium hydroxide). The mixture so formed was stirred until a clear solution was obtained. Diethanolamine (DEA) (20g) was melted and added to the gallate solution and the resultant solution "C" was stirred at 20°C for 10 minutes. 11.2g of a commercial silica gel, (Ludox AS40) (Reg. Trade Mark) which contains 40% by weight of silica, was further diluted with 40g of water and added to solution "C" over a period of 15 minutes with vigorous stirring which was continued for a further 20 minutes. The resultant gel, composition 3.55 $Na_2O$:32.4 DEA : $Ga_2O_3$:12.5 $SiO_2$:850 $H_2O$, was transferred to a stainless steel pressure vessel and crystallised at 175°C for 72 hours. The product was filtered, washed and dried at 90°C.

The product was found by X-ray powder diffraction to be crystalline with a diffraction pattern similar to and consistent with the mordenite structure.

The product was also examined by $^{29}Si$ magic angle spinning NMR and the results were similar to those in Example 1.

Example 3 - n-Butane Conversion

The gallosilicate from Example 2 was calcined at 550°C for 20 hours in a stream of air and then refluxed with a 1 molar ammonium nitrate solution (40 ml per 1g of zeolite) for 6 hours. The mixture was filtered and the filter-cake washed with deionised water, dried and calcined at 550°C for 4 hours in air. The powder was formed into pellets which were crushed and sieved to pass 12 to 30 mesh (BSS).

The catalyst (5 ml) was loaded into a reactor and activated by heating at 550°C for 4 hours in air. The reactor was then flushed with nitrogen and n-butane was passed over the activated catalyst at 550°C and 1 bar pressure (WHSV = 3 $h^{-1}$). The results obtained are given in Table 3.

Example 4 - Benzene Alkylation

The activated catalyst produced as described in Example 3 was tested with a feed of benzene and methanol (2:1 molar) at 400°C and a liquid hourly space velocity (LHSV) of 2 $h^{-1}$. The results obtained are given in Table 4.

Example 5 - Toluene Alkylation

The activated catalyst produced as described in Example 3 was tested with a feed of toluene and methanol (2:1 molar) at 400°C and an LHSV of 2 $h^{-1}$. The results obtained are given in Table 5.

Table 3

n-Butane Conversion

| n-Butane Conversion wt % | Yield - wt % | | |
|---|---|---|---|
| | $C_1$-$C_3$ aliphatics | Greater than $C_9$ | Aromatics |
| 92.2 | 38.4 | 5.0 | 40.0 |

### Table 4
### Methanol Alkylation

Feed : Benzene/methanol (2:1 molar)
Temperature : 400°C
LHSV : 2 $h^{-1}$

| Product Composition | (wt %) |
|---|---|
| Methanol | less than 0.1 |
| Benzene | 68.6 |
| Toluene | 19.8 |
| Ethylbenzene | 2.6 |
| Xylenes | 6.3 |

### Table 5
### Toluene Alkylation

Feed : Toluene/methanol (2:1 molar)
Temperature : 400°C
LHSV : 2 $h^{-1}$

| Product Composition | (wt %) |
|---|---|
| Methanol | less than 0.1 |
| Benzene | 1.9 |
| Toluene | 71.1 |
| Ethylbenzene | 0.3 |
| Xylenes | 19.6 |

Claims:

1. A crystalline gallosilicate having the following molar oxide composition:

$$0.9 \pm 0.25\ M_{2/n}O:Ga_2O_3:xSiO_2:yH_2O:zQ$$

wherein M is at least one cation which has a valency of n, x is at least 3, $H_2O$ is water of hydration additional to water notionally present when M is H, y/x is from 0 to 5, Q is a template used in the synthesis of the gallosilicate, and z is 0-20, and wherein the gallosilicate in the calcined hydrogen-form has an X-ray diffraction pattern substantially as set forth in Table A of the specification.

2. A gallosilicate according to claim 1 wherein the gallosilicate product or the hydrogen-form thereof is ion-exchanged, mixed and/or impregnated with compounds of any one of the metals or groups of metals belonging to Groups IB, IIB, IIIA, IVA, VA, VIB, VIIB and VIII according to the Periodic Table due to Mendeleef.

3. A gallosilicate according to claim 2 wherein the metals or groups of metals are selected from copper, silver, zinc, aluminium, gallium, indium, thallium, lead, antimony, bismuth, chromium, molybdenum, tungsten, manganese, iron, cobalt, nickel, ruthenium, rhodium, palladium, iridium, platinum, rhenium, thorium and the rare earth metals.

4. A process for producing gallosilicates claimed in any one of the preceding claims 1 to 3 said process comprising forming an initial mixture containing a source of silica, a source of gallia, a source of alkali metal(s), water and a template, mixing them together while maintaining the mixture at a temperature from 0 to 100°C until a homogeneous gel is formed and crystallising the gel so-formed at a temperature above 70°C, for a period of at least 2 hours.

5. A process according to claim 4 wherein the silica to gallia mole ratio in the initial mixture is at least 3:1, the free alkali(s) hydroxide to water mole ratio, defined as:

$$\frac{[(\text{Number of moles of total alkali metal(s)}) - (\text{Number of moles of alkali metal(s) required to convert gallia present to alkali metal gallate(s), i.e. } MGaO_2)]}{\text{Number of moles of water present}}$$

is greater than 1 x $10^{-5}$:1, the mole ratio of silica to free alkali metal(s) hydroxide is greater than 1, and the mole ratio of water to silica is less than 500:1.

6. A process according to claims 4 or 5 wherein the source of silica is selected from sodium silicate, silica hydrasol, silica gel, silica sol and silicic acid.

7. A process according to any one of the preceding claims 4 to 6 wherein the source of gallia is an inorganic gallium salt, gallium oxide, gallium hydroxide or gallates.

8. A process according to any one of the preceding claims 4-7 wherein the source of gallium is gallium hydroxide activated by the addition of an aqueous alkali hydroxide and a template to an appropriate proportion of freshly precipitated gallium hydroxide and then adding the source of silica slowly to the activated gallium hydroxide.

9. A process according to any one of the preceding claims 4 to 8 wherein the template is selected from one or more of the following:

(a) an alkene diamine or a dialkenetriamine or a polyalkylene polyamine,

(b) an alkanolamine selected from mono-, di- and tri-alkanol amine and in which the alkanol group is derived from ethanol, propanol or butanol,

(c) ammonia, and

(d) an alkylene glycol, a $C_1$-$C_6$ polyol or a polyoxyalkylene glycol in which the alkylene groups is ethylene, propylene or butylene.

10. Gallosilicates as claimed in anyone of the preceding claims whenever used, whether or not impregnated and/or ion-exchanged, admixed, supported or bound with other metal compounds, as catalysts for any of the following reactions: alkylation, dealkylation or transalkylation of aromatics; dehydrocyclodimerisation; aromatisation; isomerisation; dehydrogenation; hydrogenation; cracking; hydrocracking; cyclisation; oligomerisation; polymerisation; etherification; dehydration; and conversion of carbon monoxide/hydrogen mixtures to hydrocarbon.